# EUROPEAN PATENT SPECIFICATION

(11) **EP 4 215 169 B1**
(45) Date of publication and mention of the grant of the patent: **03.06.2026**
(21) Application number: 23151679.0
(22) Date of filing: 16.01.2023
(51) Int. Cl.: A61F 7/00, A61F 7/02

(54) **COLD/HOT COMPRESS DEVICE WITH EFFICIENT COLD/HOT SWITCH AND QUICK COMPRESS STRIP REPLACEMENT**
KALT-/HEISSKOMPRESSENVORRICHTUNG MIT EFFIZIENTEM KALT-/HEISSSCHALTER UND SCHNELLKOMPRESSENSTREIFENERSATZ
DISPOSITIF DE COMPRESSE FROIDE/CHAUDE AVEC COMMUTATEUR FROID/CHAUD EFFICACE ET REMPLACEMENT DE BANDE DE COMPRESSE RAPIDE

(30) Priority: 24.01.2022 TW 111102994
(43) Date of publication of application: 26.07.2023
(73) Proprietor: Hwang, Chin-Hwa, Taipei City (TW)
(72) Inventor: Hwang, Chin-Hwa, Taipei City (TW)
(74) Representative: Zeitler Volpert Kandlbinder Patentanwälte

(56) References cited:
- US-A- 3 967 627
- US-A- 5 344 436
- US-A1- 2004 068 309
- US-A1- 2018 369 015
- US-A1- 2020 008 975

## Description

### TECHNICAL FIELD OF THE DISCLOSURE

The present disclosure relates to a cold/hot compress device, and more particularly to a cold/hot compress strip that separates a cooling source and a heating source from each other, therefore not only saving energy, but also allowing users to quickly replace the compress strip for different affected parts of a user as needed.

### DESCRIPTOIN OF RELATED ART

Most of the current cold/hot compress strips use an thermoelectric cooling chip as a temperature generating source, and the principle is that after an N-type semiconductor and a P-type semiconductor on the thermoelectric cooling chip are electrically conducted, current flows from the N-type semiconductor to the P-type semiconductor to absorb heat energy to form a cooling end. If the current is switched to a reverse direction, the current flowing from the P-type semiconductor to the N-type semiconductor will release heat energy to form a heating end, so that the thermoelectric cooling chip can achieve both cooling and heating functions.

The cold/hot compress strip usually uses cold water or hot water as a cold/hot conduction medium of the thermoelectric cooling chip, so that the cold water and the hot water can circulate in a preset pipeline to achieve the cold/hot compress effect. For example, a massage device as disclosed in R.O.C. Pat. No. I614012 mainly includes a water pump and an thermoelectric cooling chip installed in a water tank, and a plurality of tubes communicating with a water bag, such that the thermoelectric cooling chip can control the circulating water in the water tank to be heated or refrigerated to a predetermined temperature, and the circulating water pumped by the water pump from the water tank into the water bag through the plurality of tubes, and then recirculated into the water tank. Therefore, the circulating water in the water bag can maintain a constant temperature and achieve the cold/hot compress effect when the water bag is placed against the skin of a user.

In the aforementioned prior art, both cold and hot compress functions are achieved by using the same thermoelectric cooling chip, the same circulation pipeline and the same water tank, and when the cold compress is switched to the hot compress function, it is necessary to heat the cold water/ice water to have the hot compress effect. Similarly, when the hot compress is switched to the cold compress, it is necessary to cool the warm/hot water to produce cold/ice water to have the cold compress effect. Obviously the heating and cooling processes are slow and inefficient and consume much power and waste energy. Further relevant prior art is disclosed in US 2018/369015 A1, US 2004/068309 A1, US 2020/008975 A1, US 5 344 436 A and US 3 967 627 A.

In view of the aforementioned drawbacks of the related art, the present discloser based on years of experience in the related industry to conduct research and experiment, and finally provided a cold/hot compress device with efficient cold/hot switch and quick compress strip replacement to improve the drawbacks of the conventional cold/hot compress device that uses the same cooling and heating sources and the same circulation pipeline, thus resulting in low efficiency and wasting energy.

### SUMMARY OF THE INVENTION

The invention is limited by the scope of independent claim 1. Further embodiments are disclosed in the dependent claims. Methods of treatment and use by the device are disclosed for the purpose of illustrating the device and are not claimed.

### SUMMARY OF THE DISCLOSUE

It is a primary objective of this disclosure to disclose a cold/hot compress device with efficient cold/hot switch and quick compress strip replacement, which separates the cooling source and the heating source from each other to improve the efficiency of switching to a cold/hot compress temperature. This disclosure not only saves energy, but also allows users to quickly replace the compress strip for different affected parts as needed.

To achieve the aforementioned and other objectives, this disclosure provides a cold/hot compress device with efficient cold/hot switch and quick compress strip replacement, which includes a host machine, and a flexible compress strip detachably connected to the host machine and attachable to a human body surface.

The host machine includes a container for containing cold water, a cooling chip installed above the container and having a cooling surface and a heat dissipation surface, a thermal conduction frame stacked against the cooling surface of the cooling chip and extended into the container for chilling the cold water, a heat dissipation module installed on the heat dissipation surface of the cooling chip, a water pump for circulating and pumping the cold water, and a control unit for controlling the operation of the host machine and the compress strip.

The compress strip is formed by stacking and superimposing two flexible substrates on each other, and a water path between the two substrates is provided for circulating the cold water, and a plurality of tubes is disposed between the water path and the water pump of the host machine and communicated with each other, and an outer surface of one of the substrates is provided with a heating film isolated from the water path, and a power cable is installed and electrically connected between the heating film and the host machine.

The host machine includes a water valve for receiving a control instruction from the control unit to control the ON/OFF state of the tubes, so that the water pump can pump the cold water contained in the container and cooled by the cooling chip through the plurality of tubes to the water path in the compress strip for circulation in order to perform a cold compress; or empty the cold water in the water path of the compress strip back into the container through the water pump and the water valve, and use the control unit to control the heating film to generate heat to perform a hot compress.

In an embodiment, a connection module is installed between the host machine and the compress strip for conveniently installing and removing the compress strip, and the connection module includes a plurality of sockets for socketing the plurality of tubes to communicate the host machine with the compress strip, and an electrical connector is provided for electrically connecting a power cable between the host machine and the compress strip.

Compared with the prior art, this disclosure separates the cooling source and the heating source from each other. In use, the cooling chip is used to cool the cold water and drive the cold water to circulate to the compress strip for a cold compress. For a hot compress, the cold water contained in the compress strip is drawn out and emptied, and the heating film generates heat to perform a hot compress, and such arrangement can prevent the cold water contained in the compress strip from being heated which results in a waste of energy. This disclosure overcomes the drawbacks of the prior art including the repeated cooling and heating of the circulating water and resulting in a poor cold and hot switch efficiency and a waste of energy. In other words, the disclosure can achieve an efficient cold/hot switch to save energy. In addition, the connection module installed between the host machine and the compress strip allows users to quickly replace the compress strip with an appropriate one according to the user's different affected parts as needed, and the application is simple and practical.

The technical characteristics of this disclosure will become apparent with the detailed description of preferred embodiments accompanied with the illustration of related drawings.

### BRIEF DESCRIPTION OF THE DRAWINGS

FIG. 1 is a perspective view of this disclosure;
FIG. 2 is an exploded view of a host machine of this disclosure;
FIG. 3 is a cross-sectional view of a host machine of this disclosure;
FIG. 4 is an exploded view of a compress strip of this disclosure;
FIG. 5 is a schematic view showing the heat dissipation of a heat dissipation module of this disclosure;
FIG. 6 is a schematic view showing the heat dissipation of a heat dissipation module in accordance with a second embodiment of this disclosure;.
FIG. 7 is a schematic view showing the heat dissipation of a heat dissipation module in accordance with a third embodiment of this disclosure; and
FIG. 8 is a perspective view of a host machine in accordance with another embodiment of this disclosure.

### DETAILED DESCRIPTION OF THE DISCLOSURE

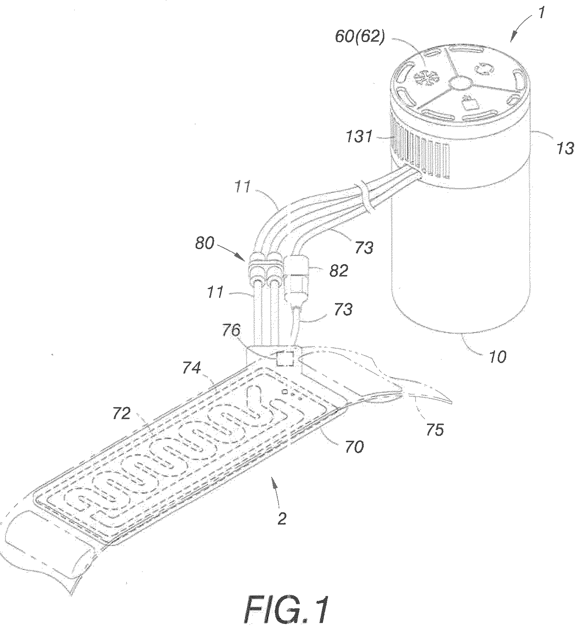

With reference to FIG. 1 for a cold/hot compress device with efficient cold/hot switch and quick compress strip replacement in accordance with this disclosure, the cold/hot compress device includes a host machine 1, and a compress strip 2 detachably connected to the host machine 1, and the compress strip 2 is a flexible sheet that can be adhered to an affected part on a human body surface.

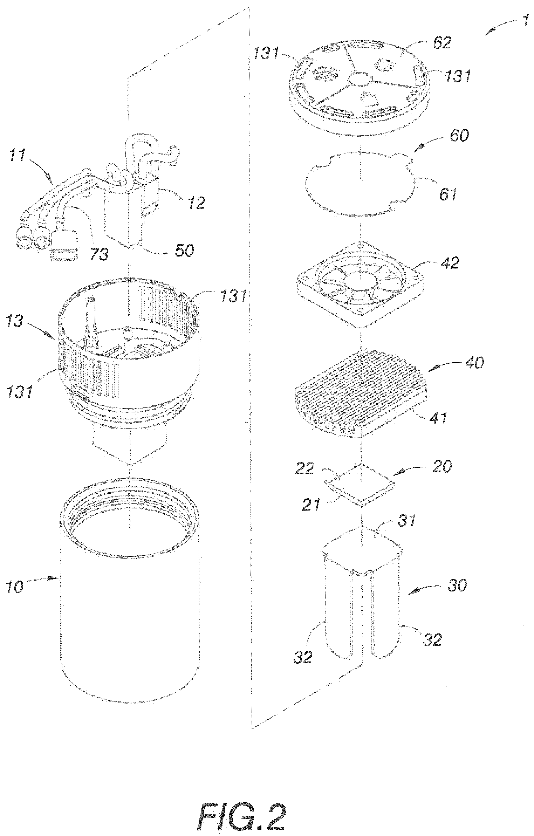

In FIGS. 2 to 4, the host machine 1 includes a container 10 for containing cold water , a cooling chip 20 installed at a position above the container 10 and having a cooling surface 21 and a heat dissipation surface 22, a thermal conduction frame 30 stacked against the cooling surface 21 of the cooling chip 20, a heat dissipation module 40 installed on the heat dissipation surface 22 of the cooling chip 20, a water pump 50 for circulating and pumping cold water, and a control unit 60 for controlling the operation of the host machine 1 and the compress strip 2.

The compress strip 2 is formed by stacking and superimposing two flexible substrates 70 with each other by high temperature welding, and a water path 71 between the two substrates 70 is provided for circulating the cold water, and the outer surface one of the substrates 70 is provided with a heating film 72 isolated from the water path 71, and a power cable 73 is installed and electrically connected between the heating film 72 and the host machine 1

In an embodiment, the two substrates 70 are soft plastic films or thin films, and the water path 71 is curved and coiled between the two substrates 70, and the heating film 72 is made of a flexible material, having a shape corresponding to the gap of the water path 71, and arranged in a staggered manner.

The water path 71 has a water inlet 711 and a water outlet 712, and a plurality of tubes 11 is installed between the water inlet 711/the water outlet 712 and the water pump 50 and communicated with the container 10, and the host machine 1 has a water valve 12 controlled by the control unit 60, and the water valve 12 is used for turning on/off the plurality of tubes 11 to allow the cold water contained in the container 10 to be pumped by the water pump 50 and passed through the water valve 12, the tube 11, and the water inlet 711 into the water path 71 of the compress strip 2, and then returned from the water outlet 712, and the tube 11 back into the container 10 to define a water cycle, or the cold water in the water path 71 of the compress strip 2 is pumped through the water valve 12 and the tube 11 back into the container 10.

When a user attaches the compress strip 2 to an affected part on a human body surface, the cooling surface 21 of the cooling chip 20 absorbs the heat energy of the cold water contained in the container 10 through the thermal conduction frame 30, such that after the cold water is cooled and refrigerated by the cooling chip 20 and the thermal conduction frame 30, the cooled cold water is pumped by the water pump 50 of the host machine 1 to pass through the plurality of tubes 11 and circulate to the water path 71 in the compress strip 2, so as to allow the compress strip 2 attached to the affected part of the human body surface to perform the cold compress.

When it is necessary to have a hot compress, the host machine 1 can quickly draw out the cold water in the water path 71 of the compress strip 2 by the water pump 50 and the water valve 12 and pumps the cold water back into the container 10 to prevent the cold water from returning into the compress strip 2, and then the control unit 60 controls the heating film 72 to generate heat, so as to allow the compress strip 2 attached to the affected part on the human body surface to perform a hot compress. In an embodiment, the control unit 60 controls the voltage supplied to the heating film 72 to generate heat at different temperatures, and allow users to adjust the hot compress temperature as required.

With the above structure, this disclosure separates the cooling source and the heating source from each other. In a cold compress, the components such as the cooling chip 20 and the water pump 50 can be used to pump the cooled cold water into the water path 71 of the compress strip 2 for a cold/ hot compress. In this case, the cold water in the water path 71 of the compress strip 2 can be drawn out first, and then the heating film 72 can be used to generate heat for the hot compress, and prevent the cold water from being heated in the compress strip 2 or consuming the heat energy generated by the heating film 72. Therefore, the cold/hot compress can be switched in an efficient, cost-effective, and power-saving manner without requiring the repeated cooling and heating of the water like the prior art. In actual implementations, if the compress strip 2 is used in a low-temperature environment, for example, the room temperature is lower than the water temperature for a cold compress, the heating film 72 may be turned on to increase the cold water temperature during the circulation of the cold water.

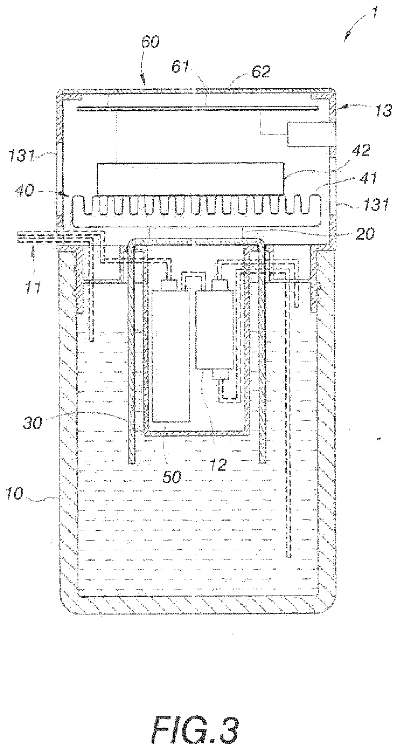

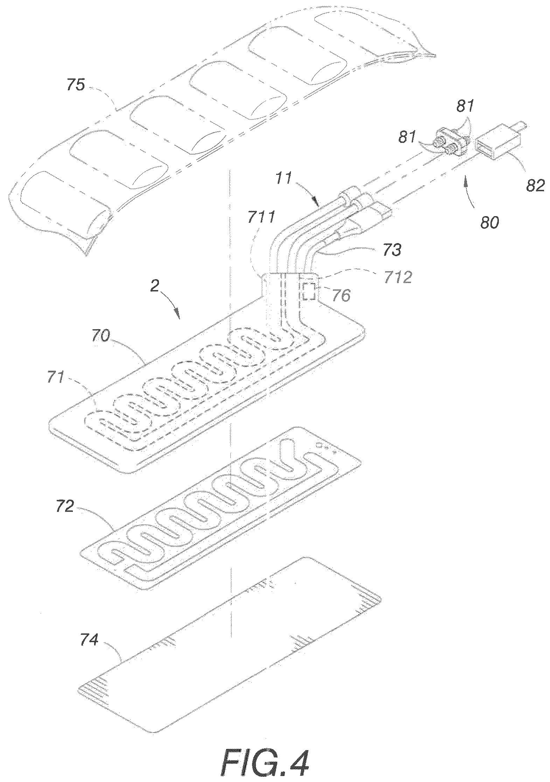

In FIGS. 3 and 4, the host machine 1 and the compress strip 2 can be connected, installed or removed directly through the plurality of tubes 11 and the power cable 73, or a connection module 80 is further provided for the connection, installation or removal in order to facilitate users to quickly replace the compress strip 2 according to different affected parts as needed. Wherein, the connection module 80 includes a plurality of sockets 81, and an electrical connector 82, and the plurality of sockets 81 is provided for quickly socketing the plurality of tubes 11 to connect the host machine 1 with the compress strip 2. The electrical connector 82 is provided for electrically connecting a power cable 73 between the host machine 1 and the compress strip 2, so that the host machine 1 can supply the electric power required for the operation of the compress strip 2. With the pluggable connection module 80, a convenient connection function can be provided for replacing the compress strip 2 with a different size to fit a different affected part of a human body, and the application is simple and practical. In an embodiment, the electrical connector 82 can be a USB port.

Other embodiments of the major components of the host machine 1 and the compress strip 2 are described as follows:

In FIGS. 2 and 3, the thermal conduction frame 30 includes a first conducting plate 31 attached to the cooling surface 21 of the cooling chip 20, and a plurality of second conducting plates 32 bent from an edge of the first conducting plate 31 and extended into the container 10, wherein the plurality of second conducting plates 32 has a fixed spacing from one another, and a space from the first conducting plate 31 is formed for fixing and installing the water pump 50 or the water valve 12.

When the user carries the host machine 1 out, the user can just carry an empty container 10 first in order to reduce weight. When a cold/hot compress is needed, the user can obtain and put room-temperature water, cold water or ice water into the container 10 conveniently, and then the cooling chip 20 and the thermal conduction frame 30 can absorb the heat of the room temperature water or the cold water to lower the temperature, wherein the container 10 can be made of a thermal insulation material to prevent the water temperature from being disturbed by the ambient temperature, assist in maintaining the cold water temperature, and save energy.

In an embodiment, the host machine 1 includes a casing 13 detachably assembled to the top of the container 10 and used for isolating the cold water from the electronic component in the host machine 1 and the cold water contained in the container 10, and covering the exterior of the heat dissipation module 40.

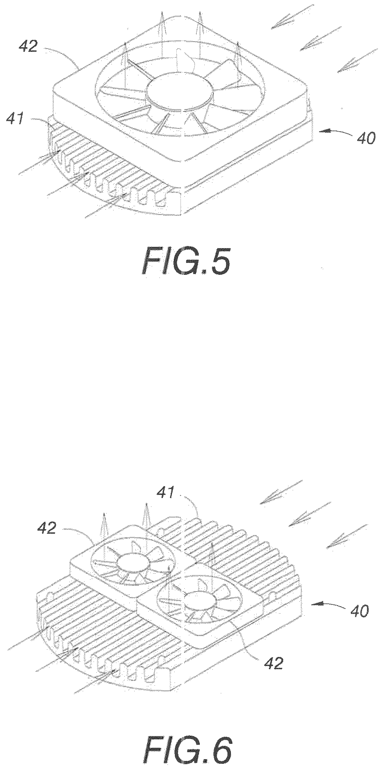

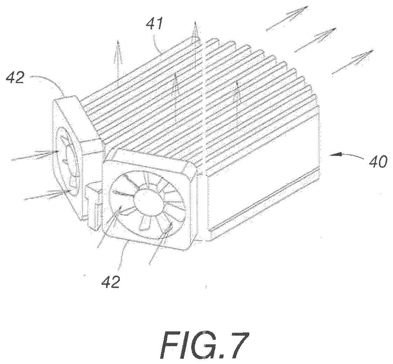

The heat dissipation module 40 includes a plurality of cooling fins 41 attached to the heat dissipation surface 22 of the cooling chip 20, and a fan 42 for blowing to cool the plurality of cooling fins 41, and the casing 13 has a plurality of through holes 131 formed around the fan 42, such that the waste heat generated during the operation of the cooling chip 20 can be dissipated by the heat dissipation module 40 and the fan 42 into the surrounding environment. In an embodiment, the fan 42 can have different designs according to the size and function of the host machine 1. As shown in FIG. 5, there is one fan 42 mounted at a position above the plurality of cooling fins 41, or as shown in FIG. 6, there are two fans 42 mounted at positions above the plurality of cooling fins 41 respectively, or as shown in FIG. 7, there are two fans 42 installed on both sides of the plurality of cooling fins 41 respectively.

In FIGS. 1 to 3, the control unit 60 includes a circuit board 61, a wireless transmission module (not shown in the figure) provided for a signal transmission with an external mobile device, an operating interface 62 installed on a surface of the casing 13 for operating a cold/hot temperature, and a battery (not shown in the figure) for supplying the electric power required for the operation of the host machine 1 and the compress strip 2. The users can directly control the cooling/heating time and temperature by the operating interface 62 of the control unit 60, or by the signal connection of the wireless transmission module and the external mobile device to facilitate the users to set the cooling/heating time and temperature through the external mobile device.

In the figures, the circuit board 61 is installed at a position above the heat dissipation module 40. In an embodiment, the distance of the circuit board 61 from the heat dissipation module 40 can be fixed according to a certain design. For example, when the fan 42 is installed above the plurality of cooling fins 41, it is necessary to maintain a specific distance between the fan 42 and the circuit board 61 to ensure that sufficient air can flow into the casing 13, and when the fan 42 is installed on the side of the cooling fins 41, the height of the cooling fins 41 can be maximized in order to be closer to the circuit board 61

In FIGS. 1 and 4, the exterior of one of the substrates 70 of the compress strip 2 is further provided with a thermoconductive layer 74, and the thermoconductive layer 74 is laid on the water path 71 and an outer surface of the heating film 72 and attached to human body, and the cold water in the water path 71, or the heat of the heating film 72 can be uniformly conducted to an affected part of human body. In an embodiment, the thermoconductive layer 74 is made of a flexible graphite sheet, or a flexible hydrogel sheet with a high thermal conductivity.

In addition, the exterior of the other substrate 70 of the compress strip 2 is provided with a fastener 75 such as a hook and loop fastener , a buckle strap or a buckle strap with a plurality of pressurized air bags, so that the compress strip 2 can be tied and fixed to the affected part of the user's body during use, wherein the buckle strap with the plurality of pressurized air bags provides an elastic compression effect to attach the aforementioned thermoconductive layer 74 more closely to the affected part to enhance the cold/hot compress effect.

In addition, the compress strip 2 of an embodiment further includes a temperature sensing module 76 capable of sensing temperature, and the temperature sensing module 76 can sense a temperature value to assure that the compress strip 2 has achieved the predetermined cold/hot temperature. It is noteworthy that the temperature sensing module 76 can also be installed on the host machine 1 in other embodiments.

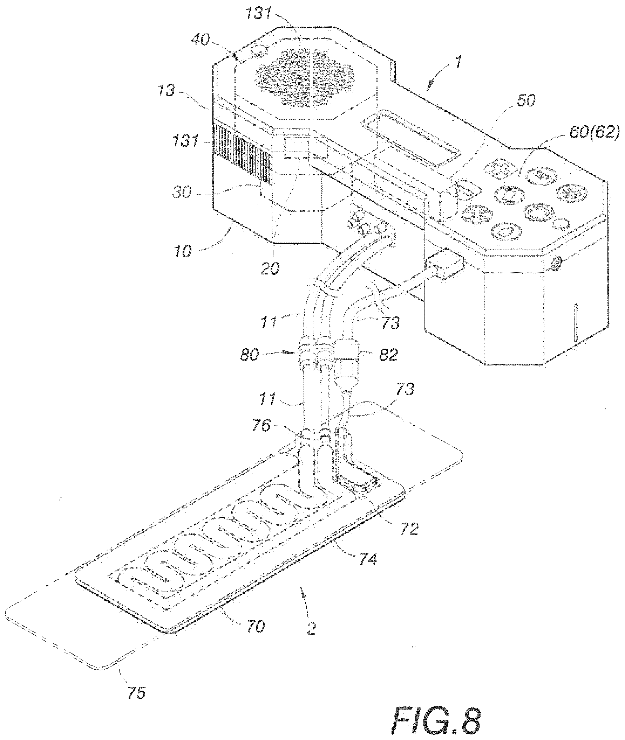

In an embodiment of the host machine 1, the control unit 60 may have different configurations as shown in FIGS. 1 and 3, and the host machine 1 is in a cylindrical shape, and its control unit 60 is installed at the top of the container 10, cooling chip 20, thermal conduction frame 30, heat dissipation module 40 and water pump 50, or as shown in FIG. 8, the host machine 1 is in the shape of an elongated polygonal box with two ends and a middle section disposed between the two ends. The container 10, cooling chip 20, thermal conduction frame 30 and heat dissipation module 40 are installed at one end of the host machine 1, and the control unit 60 is installed at the other end of the host machine 1, and the water pump 50 is installed at a position in the middle section.

While the disclosure has been described by means of specific embodiments, numerous modifications and variations could be made thereto by those skilled in the art without departing from the scope of the invention as set forth in the claims.

## Claims

1. A cold/hot compress device with efficient cold/hot switch and quick compress strip replacement, comprising a host machine (1), and a flexible compress strip (2) detachably coupled to the host machine (1) and attachable to a human body surface,
wherein the host machine (1) comprises a container (10) for containing cold water, a cooling chip (20) installed at the top of the container (10) and having a cooling surface (21) and a heat dissipation surface (22), a thermal conduction frame (30) stacked against the cooling surface (21) of the cooling chip (20) and extended into the container (10) for cooling the cold water, a heat dissipation module (40) installed on the heat dissipation surface (22) of the cooling chip (20), a water pump (50) for circulating and pumping the cold water, and a control unit (60) for controlling the operation of the host machine (1) and the compress strip (2);
the compress strip (2) is formed by stacking and superimposing two flexible substrates (70) on each other, and a water path (71) between the two substrates (70) is provided for circulating the cold water, and a plurality of tubes (11) is disposed between the water path (71) and the water pump (50) of the host machine (1) and communicated with each other, and an outer surface of one of the substrates (70) is provided with a heating film (72) isolated from the water path (71), and a power cable (73) is installed and electrically coupled between the heating film (72) and the host machine (1); and
the host machine (1) is provided with a water valve (12) for receiving a control instruction from the control unit (60) to control the ON/OFF state of the tubes (11), so that the water pump (50) can pump the cold water contained in the container (10) and cooled by the cooling chip (20) through the plurality of tubes (11) to the water path (71) in the compress strip (2) for circulation to perform a cold compress; and
emptying the cold water in the water path (71) of the compress strip (2) back into the container (10) through the water pump (50) and the water valve (12), and use the control unit (60) to control the heating film (72) to generate heat to perform a hot compress;
and **characterized in that** the other substrate (70) of the compress strip (2) is further provided with a fastener (75) on the outside thereof and disposed on another side opposite to the thermoconductive layer (74), and the fastener (75) is capable of tying and fixing the compress strip (2) to the affected part of a human body, wherein the fastener (75) is a buckle strap with a plurality of pressurized air bags;
wherein the thermal conduction frame (30) of the host machine (1) comprises a first conducting plate (31) attached to the cooling surface (21) of the cooling chip (20), wherein the thermal conduction frame (30) of the host machine (1) comprises a plurality of second conducting plates (32) separately bent from an edge of the first conducting plate (31) and extended into the container (10) and having a fixed spacing formed between one another, and a space formed with the first conducting plate (31) for fixing and installing the water pump (50) or the water valve (12).

2. The cold/hot compress device with efficient cold/hot switch and quick compress strip replacement according to claim 1, wherein the two substrates (70) of the compress strip (2) are soft plastic films or thin films, and stacked and welded to each other by a high-temperature welding technology, such that the water path (71) is curved and coiled between the two substrates (70), and the water path (71) has a water inlet (711) and a water outlet (712) communicating with the plurality of tubes (11) respectively, and the heating film (72) is in a shape corresponding to the shape of a gap of the water path (71) and arranged in a staggered manner.

3. The cold/hot compress device with efficient cold/hot switch and quick compress strip replacement according to claim 2, wherein one of the substrates (70) of the compress strip (2) is further provided with a thermoconductive layer (74) disposed on the outside thereof and laid on the water path (71) and an outer surface of the heating film (72), and the thermoconductive layer (74) is capable of uniformly conducting the cold/hot temperature generated by the water path (71) or the heating film (72) to an affected part of a human body.

4. The cold/hot compress device with efficient cold/hot switch and quick compress strip replacement according to claim 3, wherein the compress strip (2) further comprises a temperature sensing module (76) capable of sensing a temperature.

5. The cold/hot compress device with efficient cold/hot switch and quick compress strip replacement according to claim 1, wherein the host machine (1) and the compress strip (2) have a connection module (80) installed therebetween and capable of quickly installing or removing the compress strip (2), and the connection module (80) comprises a plurality of sockets (81) for socketing the plurality of tubes (11) to communicate the host machine (1) with the compress strip (2), and an electrical connector (82) provided for electrically connecting a power cable (73) between the host machine (1) and the compress strip (2).

6. The cold/hot compress device with efficient cold/hot switch and quick compress strip replacement according to claim 1, wherein the host machine (1) comprises a casing (13) detachably assembled to the top of the container (10) and covering the exterior of the heat dissipation module (40), and the heat dissipation module (40) comprises a plurality of cooling fins (41) attached to the heat dissipation surface (22) of the cooling chip (20), and a fan (42) for blowing to cool the plurality of cooling fins (41), and the casing (13) is provided with a plurality of through holes (131) formed around the fan (42).

7. The cold/hot compress device with efficient cold/hot switch and quick compress strip replacement according to claim 6, wherein the control unit (60) comprises a circuit board (61), an operating interface (62) installed on a surface of the casing (13) for operating the cold/hot temperature, and a battery for supplying an electric power required for the operation of the host machine (1) and the compress strip (2).

8. The cold/hot compress device with efficient cold/hot switch and quick compress strip replacement according to claim 7, wherein the control unit (60) further comprises a wireless transmission module provided for a signal transmission with an external mobile device.

## Patentansprüche

1. Eine Kalt-/Warmkompressionsvorrichtung mit effizientem Kalt-/Warmschalter und schnellem Austausch des Kompressionsstreifens, aufweisend eine Host-Maschine (1) und einen flexiblen Kompressionsstreifen (2), der abnehmbar mit der Hauptmaschine (1) verbunden und an einer Körperoberfläche eines Menschen angebracht ist, wobei die Hauptmaschine (1) einen Behälter (10) zur Aufnahme von kaltem Wasser, einen an der Oberseite des Behälters (10) angeordneten Kühlchip (20) und mit einer Kühlfläche (21) sowie einer Wärmeableitungsfläche (22) ausgestattet ist, einen Wärmeleitrahmen (30), der in Form einer Schichtstruktur mit der Kühlfläche (21) des Kühlchips (20) übereinander angeordnet ist und sich in den Behälter (10) hinein erstreckt, um das kalte Wasser zu kühlen, ein Wärmeableitungsmodul (40), das auf der Wärmeableitungsfläche (22) des Kühlchips (20) angeordnet ist, eine Wasserpumpe (50) zum Umwälzen und Pumpen des kalten Wassers und eine Steuereinheit (60) zum Steuern des Betriebs der Hauptmaschine (1) und des Kompressionsstreifens (2);
wobei der Kompressionsstreifen (2) durch Übereinanderlegen und Aufeinandersetzen von zwei flexiblen Substraten (70) ausgebildet ist, wobei ein wasserführender Kanal (71) zwischen den beiden Substraten (70) zum Zirkulieren des kalten Wassers ausgebildet ist, und wobei mehrere Rohre (11) zwischen dem wasserführenden Kanal (71) und der Wasserpumpe (50) der Hauptmaschine (1) angeordnet und miteinander verbunden sind, und eine Außenfläche eines der Substrate (70) mit einer vom wasserführenden Kanal (71) isolierten Heizfolie (72) zur Verfügung gestellt ist, und ein Stromkabel (73) zwischen der Heizfolie (72) und der Hauptmaschine (1) angeordnet und elektrisch verbunden ist; und
wobei die Hauptmaschine (1) ein Wasserventil (12) aufweist, um einen Steuerbefehl von der Steuereinheit (60) zum Steuern des Ein-/Aus-Zustands der Rohre (11) zu empfangen, sodass die Wasserpumpe (50) das im Behälter (10) enthaltene und durch den Kühlchip (20) gekühlte kalte Wasser durch die mehreren Rohre (11) in den wasserführenden Kanal (71) im Kompressionsstreifen (2) pumpt, um es dort zirkulieren zu lassen und eine Kältekompression durchzuführen; und
um das kalte Wasser im wasserführenden Kanal (71) des Kompressionsstreifens (2) über die Wasserpumpe (50) und das Wasserventil (12) zurück in den Behälter (10) abzulassen und die Steuereinheit (60) zu verwenden, um die Heizfolie (72) zu steuern, damit diese Wärme erzeugt, um eine heiße Kompresse durchzuführen;
und **dadurch gekennzeichnet, dass** das - andere Substrat (70) des Kompressionsstreifens (2) ferner an seiner Außenseite mit einem Befestigungselement (75) versehen ist, das auf einer der wärmeleitenden Schicht (74) gegenüberliegenden Seite angeordnet ist, und dass das Befestigungselement (75) in der Lage ist, den Kompressionsstreifen (2) an der betroffenen Stelle eines menschlichen Körpers zu verschnüren und zu befestigen, wobei der Verschluss (75) ein Schnallenband mit mehreren Druckluftkammern ist;
wobei der Wärmeleitrahmen (30) der Hauptmaschine (1) folgendes aufweist: eine erste Leitplatte (31), die an der Kühlfläche (21) des Kühlchips (20) angebracht ist, wobei der Wärmeleitrahmen (30) der Hauptmaschine (1) mehrere zweite Leitplatten (32) aufweist, die separat von einer Kante der ersten Leitplatte (31) derart gebogen sind, dass sie sich in den Behälter (10) erstrecken und einen feststehenden Abstand zueinander aufweisen, sowie einen mit der ersten Leitplatte (31) ausgebildeten Raum zum Befestigen und Anordnen der Wasserpumpe (50) oder des Wasserventils (12).

2. Kalt-/Warmkompressionsvorrichtung mit effizientem Kalt-/Warmschalter und schnellem Austausch des Kompressionsstreifens nach Anspruch 1, wobei die beiden Substrate (70) des Kompressionsstreifens (2) aus weichen Kunststofffolien oder dünnen Folien hergestellt sind und mittels einer Hochtemperatur-Schweißtechnik aufeinandergesetzt und miteinander verschweißt sind, wobei der wasserführende Kanal (71) zwischen den beiden Substraten (70) gekrümmt und gewunden verläuft, und der wasserführende Kanal (71) einen Wassereinlass (711) und einen Wasserauslass (712) aufweist, wobei die mehrere Rohre (11) jeweils miteinander verbunden sind, und die Heizfolie (72) eine Form aufweist, die der Form eines Spaltes des wasserführenden Kanals (71) entspricht, und versetzt angeordnet ist.

3. Kalt-/Warmkompressionsvorrichtung mit einem effizientem Kalt-/Warm-Umschalter und schnellem Austausch des Kompressionsstreifens gemäß Anspruch 2, wobei eines der Substrate (70) des Kompressionsstreifens (2) ferner mit einer wärmeleitenden Schicht (74) versehen ist, die an dessen Außenseite angeordnet ist und auf dem wasserführenden Kanal (71) sowie einer Außenfläche der Heizfolie (72) aufliegt, wobei die wärmeleitende Schicht (74) dazu ausgebildet ist, die durch den wasserführenden Kanal (71) oder die Heizfolie (72) erzeugte Kälte- bzw. Wärme gleichmäßig an eine beeinträchtigte Stelle des menschlichen Körpers zu leiten.

4. Kalt-/Warmkompressionsvorrichtung mit effizientem Kalt-/Warmschalter und schnellem Austausch des Kompressionsstreifens gemäß Anspruch 3, wobei der Kompressionsstreifen (2) ferner ein Temperaturerfassungsmodul (76) aufweist, das in der Lage ist, eine Temperatur zu erfassen.

5. Kalt-/Warmkompressionsvorrichtung mit effizientem Kalt-/Warmschalter und schnellem Austausch des Kompressionsstreifens gemäß Anspruch 1, wobei zwischen der Hauptmaschine (1) und dem Kompressionsstreifen (2) ein Verbindungsmodul (80) angeordnet ist, das den Kompressionsstreifen (2) schnell anordnen oder entfernen kann, wobei das Verbindungsmodul (80) mehrere Buchsen (81) zum Einstecken der mehreren Rohre (11), um die Hauptmaschine (1) mit dem Kompressionsstreifen (2) zu verbinden, sowie einen elektrischen Steckverbinder (82), der zum elektrischen Verbinden eines Stromkabels (73) zwischen der Hauptmaschine (1) und dem Kompressionsstreifen (2) zur Verfügung steht, aufweist.

6. Kalt-/Warmkompressionsvorrichtung mit effizientem Kalt-/Warm-Umschalter und schnellem Austausch der Kompressionsstreifen gemäß Anspruch 1, wobei die Hauptmaschine (1) ein Gehäuse (13) aufweist, das abnehmbar an der Oberseite des Behälters (10) angebracht ist und die Außenseite des Wärmeableitmoduls (40) bedeckt, und wobei das Wärmeableitmodul (40) mehrere Kühlrippen (41), die an der Wärmeableitfläche (22) des Kühlchips (20) angebracht sind, sowie einen Lüfter (42) zum Anblasen zur Kühlung der mehreren Kühlrippen (41) aufweist, woebi das Gehäuse (13) mit mehreren Durchgangslöchern (131) versehen ist, die um den Lüfter (42) ausgebildet sind.

7. Kalt-/Warmkompressionsvorrichtung mit effizientem Kalt-/Warmschalter und schnellem Austausch der Kompressionsstreifen gemäß Anspruch 6, wobei die Steuereinheit (60) eine Leiterplatte (61), eine an einer Oberfläche des Gehäuses (13) angeordnete Bedienoberfläche (62) zur Einstellung der Kalt-/Warmtemperatur und eine Batterie zur Versorgung mit der für den Betrieb der Hauptmaschine (1) und des Kompressionsstreifens (2) erforderlichen elektrischen Energie aufweist.

8. Kalt-/Warmkompressionsvorrichtung mit effizientem Kalt-/Warmschalter und schnellem Austausch der Kompressionsstreifen gemäß Anspruch 7, wobei die Steuereinheit (60) ferner ein drahtloses Übertragungsmodul aufweist, das zur Signalübertragung mit einem externen mobilen Gerät ausgebildet ist.

## Revendications

1. Dispositif de compresse froide/chaude avec commutateur froid/chaud efficace et remplacement rapide de la bande de compresse, comprenant une machine hôte (1) et une bande de compresse souple (2) couplée de manière amovible à la machine hôte (1) et pouvant être fixée à une surface de corps humain,
dans lequel la machine hôte (1) comprend un récipient (10) destiné à contenir de l'eau froide, une puce de refroidissement (20) installée sur le dessus du réservoir (10) et comportant une surface de refroidissement (21) et une surface de dissipation thermique (22), un cadre de conduction thermique (30) empilé contre la surface de refroidissement (21) de la puce de refroidissement (20) et s'étendant dans le récipient (10) pour refroidir l'eau froide, un module de dissipation thermique (40) installé sur la surface de dissipation thermique (22) de la puce de refroidissement (20), une pompe à eau (50) pour faire circuler et pomper l'eau froide, et une unité de commande (60) pour commander le fonctionnement de la machine hôte (1) et de la bande de compresse (2) ;
la bande de compresse (2) est formée en empilant et superposant deux substrats flexibles (70) l'un sur l'autre, et un circuit d'eau (71) entre les deux substrats (70) est prévu pour faire circuler l'eau froide, et une pluralité de tubes (11) sont disposés entre le circuit d'eau (71) et la pompe à eau (50) de la machine hôte (1) et communiquent entre eux, et une surface extérieure de l'un des substrats (70) est pourvue d'un film chauffant (72) isolé du circuit d'eau (71), et un câble d'alimentation (73) est installé et couplé électriquement entre le film chauffant (72) et la machine hôte (1) ; et
la machine hôte (1) est pourvue d'une vanne d'eau (12) destinée à recevoir une instruction de commande de l'unité de commande (60) pour commander l'état activé/désactivé des tubes (11), de sorte que la pompe à eau (50) puisse pomper l'eau froide contenue dans le récipient (10) et refroidie par la puce de refroidissement (20) à travers la pluralité de tubes (11) vers le circuit d'eau (71) dans la bande de compresse (2) et la faire circuler afin d'effectuer une compresse froide ;
et vider l'eau froide du circuit d'eau (71) de la bande de compresse (2) et la renvoyer dans le récipient (10) via la pompe à eau (50) et la vanne d'eau (12), et utiliser l'unité de commande (60) pour commander le film chauffant (72) pour générer de la chaleur afin d'effectuer une compresse chaude ;
**caractérisé en ce que**
l'autre substrat (70) de la bande de compresse (2) est en outre pourvu d'une attache (75) sur sa face extérieure et disposée sur un autre côté opposé à la couche thermoconductrice (74), et l'attache (75) est capable de lier et de fixer la bande de compresse (2) à la partie affectée d'un corps humain, dans lequel l'attache (75) est une sangle à boucle comportant une pluralité de coussins gonflables sous pression ;
dans lequel le cadre de conduction thermique (30) de la machine hôte (1) comprend une première plaque conductrice (31) fixée à la surface de refroidissement (21) de la puce de refroidissement (20), dans lequel le cadre de conduction thermique (30) de la machine hôte (1) comprend une pluralité de deuxièmes plaques conductrices (32) pliées séparément à partir d'un bord de la première plaque conductrice (31) et s'étendant dans le récipient (10) et ayant un espacement fixe formé entre elles, et un espace formé avec la première plaque conductrice (31) pour fixer et installer la pompe à eau (50) ou la vanne d'eau (12).

2. Dispositif de compresse froide/chaude avec commutateur froid/chaud efficace et remplacement rapide de la bande de compresse selon la revendication 1, dans lequel les deux substrats (70) de la bande de compresse (2) sont des films plastiques souples ou des films minces, et sont empilés et soudés l'un à l'autre par une technologie de soudage à haute température, de telle sorte que le circuit d'eau (71) soit courbé et enroulé entre les deux substrats (70), et le circuit d'eau (71) comporte une entrée d'eau (711) et une sortie d'eau (712) communiquant respectivement avec la pluralité de tubes (11), et le film chauffant (72) a une forme correspondant à la forme d'un espace du circuit d'eau (71) et est disposé de manière décalée.

3. Dispositif de compresse froide/chaude avec commutateur froid/chaud efficace et remplacement rapide de la bande de compresse selon la revendication 2, dans lequel l'un des substrats (70) de la bande de compresse (2) est en outre pourvu d'une couche thermoconductrice (74) disposée sur sa face extérieure et posée sur le circuit d'eau (71) et une surface extérieure du film chauffant (72), et la couche thermoconductrice (74) est capable de conduire uniformément la température froide/chaude générée par le circuit d'eau (71) ou le film chauffant (72) vers une partie affectée d'un corps humain.

4. Dispositif de compresse froide/chaude avec commutateur froid/chaud efficace et remplacement rapide de la bande de compresse selon la revendication 3, dans lequel la bande de compresse (2) comprend en outre un module de détection de température (76) capable de détecter une température.

5. Dispositif de compresse froide/chaude avec commutateur froid/chaud efficace et remplacement rapide de la bande de compresse selon la revendication 1, dans lequel la machine hôte (1) et la bande de compresse (2) comportent un module de connexion (80) installé entre elles et capable d'installer ou de retirer rapidement la bande de compresse (2), et le module de connexion (80) comprend une pluralité de prises (81) destinées à recevoir la pluralité de tubes (11) afin de faire communiquer la machine hôte (1) avec la bande de compresse (2), ainsi qu'un connecteur électrique (82) prévu pour relier électriquement un câble d'alimentation (73) entre la machine hôte (1) et la bande de compresse (2).

6. Dispositif de compresse froide/chaude avec commutateur froid/chaud efficace et remplacement rapide de la bande de compresse selon la revendication 1, dans lequel la machine hôte (1) comprend un boîtier (13) assemblé de manière amovible sur le dessus du récipient (10) et recouvrant l'extérieur du module de dissipation thermique (40), et le module de dissipation thermique (40) comprend une pluralité d'ailettes de refroidissement (41) fixées à la surface de dissipation thermique (22) de la puce de refroidissement (20), ainsi qu'un ventilateur (42) destiné à souffler de l'air pour refroidir la pluralité d'ailettes de refroidissement (41), et le boîtier (13) est pourvu d'une pluralité de trous traversants (131) formés autour du ventilateur (42).

7. Dispositif de compresse froide/chaude avec commutateur froid/chaud efficace et remplacement rapide de la bande de compresse selon la revendication 6, dans lequel l'unité de commande (60) comprend une carte de circuit imprimé (61), une interface de commande (62) installée sur une surface du boîtier (13) pour commander la température froide/chaude, ainsi qu'une batterie destinée à fournir l'énergie électrique nécessaire au fonctionnement de la machine hôte (1) et de la bande de compresse (2).

8. Dispositif de compresse froide/chaude avec commutateur froid/chaud efficace et remplacement rapide de la bande de compresse selon la revendication 7, dans lequel l'unité de commande (60) comprend en outre un module de transmission sans fil prévu pour une transmission de signaux avec un dispositif mobile externe.
